# EUROPEAN PATENT SPECIFICATION

(11) **EP 1 317 285 B1**
(45) Date of publication and mention of the grant of the patent: **07.10.2009**
(21) Application number: 01968007.3
(22) Date of filing: 17.08.2001
(51) Int. Cl.: A61K 39/42, G01N 33/53

(54) **METHODS FOR TREATING DEMYELINATING DISEASES**
METHODEN ZUM BEHANDELN VON DEMYELINISIERENDEN KRANKHEITEN
METHODES DE TRAITEMENT DE MALADIES DEMYENILISANTES

(30) Priority: 18.08.2000 US 226399 P
(43) Date of publication of application: 11.06.2003
(73) Proprietor: The Regents of The University of California, Oakland, CA 94607-5200 (US)
(72) Inventor: LANE, Thomas, E., Irvine, CA 92612 (US)
(74) Representative: UEXKÜLL & STOLBERG
(86) International application number: PCT/US2001/025767
(87) International publication number: WO 2002/015932

(56) References cited:
- WO-A-98/02151
- WO-A-02/085862
- WO-A-02/098346
- SORENSEN T L ET AL: "EXPRESSION OF SPECIFIC CHEMOKINES AND CHEMOKINE RECEPTORS IN THE CENTRAL NERVOUS SYSTEM OF MULTIPLE SCLEROSIS PATIENTS" JOURNAL OF CLINICAL INVESTIGATION, NEW YORK, NY, US, vol. 103, no. 6, March 1999 (1999-03), pages 807-815, XP001024087 ISSN: 0021-9738
- BALASHOV K E ET AL: "CCR5+ AND CXCR3+ T CELLS ARE INCREASED IN MULTIPLE SCLEROSIS AND THEIR LIGANDS MIP-1ALPHA AND IP-10 EXPRESSED IN DEMYELINATING BRAINLESIONS" PROCEEDINGS OF THE NATIONAL ACADEMY OF SCIENCES OF USA, NATIONAL ACADEMY OF SCIENCE. WASHINGTON, US, vol. 96, June 1999 (1999-06), pages 6873-6878, XP002936303 ISSN: 0027-8424
- SIMPSON J E ET AL: "EXPRESSION OF THE INTERFERON-GAMMA-INDUCIBLE, CHEMOKINES IP-10 AND MIG AND THEIR RECEPTOR, CXCR3, IN MULTIPLE SCLEROSIS LESIONS" NEUROPATHOLOGY AND APPLIED NEUROBIOLOGY, BLACKWELL SCIENTIFIC PUBLICATIONS, LONDON, GB, vol. 26, 1 April 2000 (2000-04-01), pages 133-142, XP002906681 ISSN: 0305-1846
- PIALI L ET AL: "THE CHEMOKINE RECEPTOR CXCR3 MEDIATES RAPID AND SHEAR-RESISTANT ADHESION-INDUCTION OF EFFECTOR T LYMPHOCYTES BY THE CHEMOKINES IP10AND MIG" EUROPEAN JOURNAL OF IMMUNOLOGY, WEINHEIM, DE, vol. 28, no. 3, March 1998 (1998-03), pages 961-972, XP001024114 ISSN: 0014-2980
- GANGUR V. ET AL.: 'Human IP-10 selectively promotes dominance of polyclonally activated and environmental antigen-driven IFN-gamma over IL-4 responses' FASEB J. vol. 12, 1998, pages 705 - 713, XP002906680
- SORENSEN T.L. ET AL.: 'Expression of specific chemokines and chemokine receptors in the central nervous system of multiple sclerosis patients' J. CLIN. INVEST. vol. 103, no. 6, March 1999, pages 807 - 815, XP001024087
- BALASHOV K.E. ET AL.: 'CCR5+ and CXCR3+ T cells are increased in multiple sclerosis and their ligands MIP-1alpha and IP-10 are expressed in demyelinating brain lesions' PROC. NATL. ACAD. SCI. USA vol. 96, June 1999, pages 6873 - 6878, XP002936303
- SIMPSON J.E. ET AL.: 'Expression of the interferon-gamma-inducible chemokines IP-10 and Mig and their receptor, CXCR3, in multiple sclerosis lesions' NEUROPATH. APPL. NEUROBIO. vol. 26, 2000, pages 133 - 142, XP002906681
- TAMARU M. ET AL.: 'Liver-infiltrating T lymphocytes are attracted selectively by IFN-inducible protein-10' vol. 12, no. 4, April 2000, pages 299 - 308, XP002906682

## Description

### BACKGROUND OF THE INVENTION

This invention relates to immunology and, more specifically to treatment of demyelinating diseases through neutralization of the chemokine IP-10.

Demyelinating diseases are a group of disorders characterized by damage to the myelin sheath that coats and insulates nerve fibers. Individuals afflicted with a demyelinating disease suffer neurologial deficits due to impaired transmission of nerve impulses caused by myelin being stripped from nerve fibers. Multiple Sclerosis, the most prominent of this group of diseases, is a neuroinflammatory, demyelinating disease of the central nervous system (CNS). MS is the leading cause of nontraumatic neurological disability among young adults in North America and affects approximately one million young adults worldwide.

MS usually affects multiple areas of white matter in the central nervous system (CNS), most frequently, the periventricular white matter, brainstem, spinal cord and the optic nerves. The disease initially destroys myelin sheaths and eventually kills oligodendrocytes creating the characteristic plaque of MS. The early development of the plaque generally correlates with perivascular inflammation followed by the migration of lymphocytes, plasma cells and macrophages into the lesion. Macrophage infiltration has been shown to be directly related to the severity of nerve fiber demyelination.

The incidence of MS and its pattern of distribution have been unchanged for decades. The disease usually presents in the from of recurrent attacks of focal or multifocal neurologic dysfunction. Attacks occur, remit, and recur, seemingly randomly over many years. Remission is often incomplete and as one attack follows another, a stepwise downward progression ensues with increasing permanent deficit. Current MS management includes interferons, corticosteroids and cytotoxic immunosuppressive agents, soften with unsatisfactory outcomes, significant side effects and a lack of impact on the chronic progression of MS. Current treatments can hasten the recovery from acute exacerbations of MS by reducing the inflammatory response, but generally do not prevent future attacks, development of additional disabilities or chronic progression of MS. Furthermore, no treatments currently exist that reverse the neurological impairments associated with demyelinating diseases by causing remyelination of damaged nerve fibers. As a result, supportive measures, symptomatic treatment and comprehensive rehabilitation remain at the core of management.
T.L. Sorensen et al. J. Clinical Investigation Vol. 103 No. 6:807-815 (March 1999) reported elevated levels of the chemokines IP-10, Mig and RANTES the respective chemokine receptors CXCR3 and CCR5 on the infiltrating lymphocytic cells in the cerebrospinal fluid of patients during MS attacks. At about the same time, K.E. Balashov et al. PNAS USA, Vol. 96:6873-6878 (June 1999) reported that CXCR3 and CCR5 positive T cells increased in blood of MS patients, whereas their ligands MIP-1α and IP-10 were expressed in MS brain lesions. In agreement with these findings, J.E. Simpson at al. Neuropathology and Applied Neurobiology Vol. 26:133-142 (April 2000) demonstrated that IFN-γ inducible IP-10 and Mig were predominantly expressed by macrophages within the plaque and by reactive astrocytes in the surrounding parenchyma in actively demyelinating lesions of MS patients; CXCR3 was expressed by T cells and by astrocytes within the plaque. However, none of these studies taught a method for the treatment of a demyelination disease by promoting remyelination of the affected neurons.

Thus, there exists a need for treatments that prevent the chronic progression and reverse the neurological impairments of MS and other demyelinating diseases. The present invention satisfies this need and provides related advantages as well.

### SUMMARY OF THE INVENTION

The invention provides use of a neutralizing agent specific for interferon inducible protein of 10 kDa (IP-10) for the preparation of a medicament for promoting remyelination in a subject affected with a demyelinating disease at chronic stage and for reducing the severity of a demyelinating disease at chronic stage in a subject including remyelinating demyelinated nerve fibers and impeding further demyelination. The neutralizing agent specific for interferon inducible protein of 10 kDa (IP-10) is selected from the group consisting of an antibody, antibody fragment or immunoadhesion against IP-10, an antisense nucleic acid to IP-10, a ribozyme specific to IP-10, and a fragment or peptidominatic of the CXCR3 receptor that binds IP 10. Administration of an effective amount of a neutralizing agent specific for interferon inducible protein of 10 kDa (IP-10) reduces the severity of a demyelinating disease by effecting the repair of demyelinating lesions in the nervous system and by impeding the ongoing demyelination associated with a demyelinating disease.

### BRIEF DESCRIPTION OF THE DRAWINGS

Figure 1 shows increased mortality in anti-IP-10 treated mice. The mice were infected intracranially with MHV and treated intraperitoneally with either anti-IP-10 or normal rabbit serum (NRS).
Figure 2 shows delayed viral clearance from the CNS in anti-IP-10 treated mice. The number of mice examined is designated ^{a}n. The data presented represents three independent studies.
Figure 3 shows the decreased level of T lymphocytes within the CNS of anti-IP-10 treated mice. Anti-IP-10 treatment of MHV infected mice resulted in a 82.3 percent decrease in infiltrating CD4+ T cells and a 70.4 percent decrease in infiltrating CD8+ T cells when compared to NRS treatment of MHV infected mice. The data are presented as a mean +/- standard error of the mean (SEM) and represents the results of two independent studies.
Figure 4 shows (A) analysis of IFN-γ mRNA transcripts in anti-IP-10 treated mice at day 7 post infection (*P≤0.05; NRS, n=3; anti-IP-10, n=3) and (B) decreased expression of IFN-γ protein levels in anti-IP-10 treated mice at day 7 post infection (*P≤0.01; NRS, n=5; anti-IP-10, n=5).
Figure 5 shows the clinical scores of MHV-infected mice treated with either anti-IP-10, anti-Mig or NRS starting at day 12 post infection and ending at day 19 days infection. Clinical disease is assessed according to a numerical scoring system based upon the following parameters: 0 - no abnormality; 2 - limp tail and partial hindlimb weakness, 3 - complete hindlimb paralysis, 4 - death.
Figure 6 shows post treatment infiltration, measured at day 28 post MHV infection, of CD4⁺ and CD8⁺ T lymphocytes into the CNS of mice treated with anti-IP-10, anti-Mig or NRS starting at day 12 post infection and ending at day 19 post infection.
Figure 7 shows post treatment infiltration, measured at day 21 post MHV infection, of CD4⁺ T lymphocytes, CD8⁺ T lymphocytes and macrophages into the CNS of mice treated with anti-IP-10, anti-Mig or NRS at day 12 post infection and ending at day 19 post infection.
Figure 8 shows demyelination at 21 days post MHV infection in mice treated with either anti-IP-10, anti-Mig or NRS starting at day 12 post infection and ending at day 19 post infection (C.S., Clinical Score, DM, Demyelination).
Figure 9 shows delayed viral clearance from the CNS in anti-Mig treated mice. The number of mice examined is designated n.
Figure 10 shows Mig expression at day 7 post infection and day 12 post infection in the CNS of MHV infected mice. The data are presented as a mean ± SEM (PID 7 n=3; PID 12 n=2; PID 35 NRS n=3)
Figure 11 shows the increased mortality in anti-Mig treated mice. For the anti-Mig group n=27 and for the NRS group n=27.
Figure 12 shows decreased levels of infiltrating T lymphocytes within the CNS of anti-Mig treated mice. The data are presented as a mean ± SEM and represent the results of two independent studies. For the NRS group n=5 and for the anti-Mig group n=5.
Figure 13 shows cytokine expression in brains of MHV infected mice treated with either anti-Mig or NRS. Data are presented as mean ± SEM (*p ≤ 0.02). For the NRS group n=3 and for the anti-Mig group n=3.
Figure 14 shows decreased expression of IFN-γ protein in anti-Mig treated mice. Data are presented as mean ± SEM (*p ≤ 0.05). For the NRS group n=5 and for the anti-Mig group n=5.
Figure 15 shows increased expression of IL-10 protein in anti-Mig treated mice. Data are presented as mean ± SEM (*p ≤ 0.02). For the NRS group n=5 and for the anti-Mig group n=5.
Figure 16 shows (A) remyelination of viral-induced nerve fiber demyelination by anti-IP 10 treatment, and (B) viral-induced nerve fiber demyelination without anti-IP 10 treatment.

### DETAILED DESCRIPTION OF THE INVENTION

This invention relates to treating demyelinating diseases including Multiple Sclerosis (MS) at chronic stage by neutralizing interferon inducible protein of 10 kDA (IP-10) with a specific neutralizing agent selected from the group consisting of an antibody, antibody fragment or immunoadhesion against IP-10, an antisense nucleic acid to IP-10, a ribozyme specific to IP-10, and in fragment or peptidominatic of the CXCR3 receptor that binds IP-10. Neutralization of IP-10 represents a significant treatment option for demyelinating diseases because it causes the remyelination of demyelinated nerve fibers, thereby reversing the neurological impairments associated with demyelination, and further impedes the nerve fiber demyelination that is responsible for the neurological impairments associated with demyelinating diseases. Significantly, neutralization of IP-10 targets a cause rather than merely the symptoms of neurological impairments associated with demyelination.

As used herein, the term "demyelinating disease" is intended to refer to a disease or condition that is characterized by the loss of the myelin sheath surrounding the nerve fibers (axons) of nerve cells. Inflammation can accompany the loss of myelin in demyelinating diseases. The clinical course of a demyelinating disease can be, for example, acute, chronic or remitting and relapsing. The loss of myelin in a demyelinating disease can occur in the central nervous system (CNS), the peripheral nervous system (PNS) or both. For example, Multiple Sclerosis (MS) is a demyelinating disease of the central nervous system (CNS) in which demyelination occurs in the white matter of the brain and spinal cord. Acute demyelinating polyneuropathy, better known as Guillain-Barre syndrome (GBS) and its variant chronic inflammatory demyelinating polyneuropathy (CIDP) are examples of demyelinating diseases of the peripheral nervous system.

As used herein, the term "neuroinflammatory disease" refers to a disease that is characterized by T-cell infiltration into the central nervous system. Neuroinflammatory conditions include both demyelinating diseases and non-demyelinating inflammatory diseases, for example, viral encephalitis, bacterial encephalitis and bacterial meningitis. Viral encephalitis can be caused by a variety of infectious agents including, for example, Japanese encephalitis virus, HIV-1 and cytomegalovirus.

As used herein, the term "remyelination" refers to a gross increase in the myelination of demyelinated nerve fibers. A gross increase in myelination can be detected through a variety of methods known in the art

As used herein, the term "effective amount" when used in reference to interferon inducible protein of 10 kDa (IP-10), is intended to mean an amount of a neutralizing agent specific for IP-10 sufficient to reduce the severity of a demyelinating disease or sufficient to promote remyelination in a subject affected with a demyelinating disease.

As used herein, "reduction in severity" is intended to refer to an arrest, decrease or reversal in signs and symptoms, physiological indicators, biochemical markers or metabolic indicators associated with a particular disease. Symptoms of a demyelinating disease include, for example, neurological impairments and neuroinflammation. Physiological indicators of a demyelinating disease include the demyelination of nerve fibers. Biochemical markers of a demyelinating disease are, for example, myelin, gamma globulin or the specific molecules that give rise to oligoclonal banding. Demyelination as well as remyelination of nerve fibers can be detected by a variety of clinical methods well known in the art. For example, evoked potentials (EP) can be used to measure how quickly nerve impulses travel along the nerve fibres in various parts of the nervous system. In addition, computer-assisted tomography (CT) can be used to scan the central nervous system to detect areas of demyelination as well as remyelination of nerve fibers. Magnetic resonance imaging (MRI) also can be used to scan the central nervous system, but without the use of x-rays. More sensitive than the CT scan, MRI can detect areas of demyelination as well as remyelination that may not be seen by the CT scanner. Moreover, lumbar puncture or spinal tap procedures can be used to draw out cerebrospinal fluid. The fluid is examined for increased levels of gamma globulin and oligoclonal banding. These and other methods well known in the art can be used to determine the severity of a demyelinating disease by measuring myelination changes such as demyelination as well as remyelination of nerve fibers.

As used herein, the term "neutralizing agent specific for interferon inducible protein of 10 kDa (IP-10)" is intended to refer to an agent effecting a decrease in the extent, amount or rate of IP-10 expression or effecting a decrease in the activity of IP-10, said agent being selected from the group consisting of an antibody, antibody fragment or immunoadhesion agains IP-10, an antisense nucleic acid to IP-10, a ribozyme specific to IP-10, and a fragment or peptidominatic of the CXCR3 receptor that binds IP 10.

The invention provides use of a neutralizing agent of the invention, specific for interferon inducible protein of 10 kDa (IP-10) for the preparation of a medicament for promoting remyelination in a subject affected with a demyelinating disease at chronic stage and reducing the severity of a demyelinating disease at chronic stage comprising remyelinating demyelinated nerve fibers and impeding further demyelination of affected nerve fibers.

A successful host response to inflammation generally requires the accumulation of specialized host cells at the site of tissue damage. This cellular accumulation is a critical step in the normal inflammatory process and is mediated by a family of secreted chemotactic cytokines that have in common important structural features and a role in pathogenic inflammation. IP-10 and Mig belong to this protein family of over forty structurally and functionally related proteins known as chemokines.

Chemokines are homologous 8 to 14 kDa heparin binding proteins that possess a conserved structural motif containing two cysteine pairs and are divided into subfamilies based on the relative position of the cysteine residues in the mature protein. There are at least four subfamilies of chemokines, but only two, α-chemokines and β-chemokines, have been well characterized. In the α-chemokines, the first two cysteine residues are separated by a single amino acid (CXC), whereas in the β-chemokines the first two cysteine residues are adjacent to each other (CC). The C-X-C chemokines include, for example, interleukin-8 (IL-8), human platelet derived factors, IP-10 and Mig. Members of the C-C chemokine subfamily include, for example, macrophage chemoattractant protein (MCP), macrophage inflammatory protein-1α (MIP-1α), macrophage inflammatory protein-1β (MIP-1β) and regulated on activation, normal T-cell expressed and secreted (RANTES).

Chemokines selectively attract leukocyte subsets; some chemokines act specifically toward eosinophils, others toward monocytes, dendritic cells, or T cells (see Luster, A., New Engl. J. Med. 338: 436-445 (1998)). In general, CC chemokines chemoattract monocytes, eosinophils, basophils, and T cells; and signal through the chemokine receptors CCR1 to CCR9. The CXC chemokine family can be further divided into two classes based on the presence or absence of an ELR sequence (Glu-Leu-Arg) near the N terminal preceding the CXC sequence. The ELR-containing CXC chemokines including IL-8 chemoattract neutrophils, while the non-ELR CXC chemokines including IP-10 and Mig chemoattract T lymphocytes.

Chemokines induce cell migration and activation, in part, by binding to specific G-protein coupled cell-surface receptors on target cells. More than 10 distinct chemokine receptors, each expressed on different subsets of leukocytes, have been identified. Chemokine receptors are constitutively expressed on some cells, whereas they are inducible on others. CXCR3, the receptor recognized by IP-10 and Mig, is expressed on activated T lymphocytes of the T helper type 1 (Th1) phenotype and Natural Killer (NK) Cells. Significantly, a central mechanism in the pathology of neuroinflammatory diseases, including both demyelinating and non-demyelinating neuroinflammatory diseases, is the organ-specific migration of activated T cells into the central nervous system.

Demyelinating diseases are an important group of neurological disorders because of the frequency with which they occur and the disability that they cause. Demyelinating diseases have in common a focal or patchy destruction of myelin sheaths that can be accompanied by an inflammatory response. Myelin loss also occurs in other conditions, for example, in genetically determined defects in myelin metabolism, and as a consequence of toxin exposure and infections of oligodendrocytes or Schwann cells.

Demyelinating diseases can be divided into processes affecting myelin of the central nervous system and those affecting myelin of the peripheral nervous system. Demyelinating diseases of the central nervous system include, for example, MS, acute disseminated encephalomyelitis (ADE) including postinfectious and postvaccinal encephalomyelitis, acute necrotizing hemorrhagic encephalomyelitis and progressive (necrotizing) myelopathy. Demyelinating diseases of the peripheral nervous sytem include, for example, acute inflammatory demyelinating polyradiculoneuropathy (Guillain-Barré syndrome), chronic inflammatory demyelinating polyradiculoneuropathy (CIDP), demyelinating neuropathy associated with IgM monoclonal gammopathy and neuropathy associated with sclerosing myeloma.

The methods of the invention can be practiced with demyelinating diseases regardless of their etiology. MS is a central nervous system demyelinating disease with a likely autoimmune etiology as reviewed in Martin et al., Annu. Rev. Immunol. 10:153-187 (1992). In contrast, ADE is a central nervous system demyelinating disease that develops following an infectious illness, often as a sequela of measles. Despite their distinct etiologies both, MS and ADE as well as other demyelinating diseases known to those skilled in the art, including those described herein are appropriate therapeutic targets for the methods of the invention based on the shared feature of inflammatory demyelination characterized by migration of lymphocytes and macrophages to the site of tissue damage. As set forth above, a central mechanism in the pathology of neuroinflammatory demyelinating diseases is the organ-specific migration of activated T cells into the central nervous system.

As described herein, IP-10 and Mig are components in mounting a host defense against inflammation of the nervous system. Specifically, as shown herein, the IP-10 and Mig coordinate the trafficking of Th1 T lymphocytes into the CNS in response to a pathological aberration such as an infectious agent or autoagressive immune cells. T lymphocytes can be subdivided into two major categories known as CD4⁺ cells and CD8⁺ cells. CD4 and CD8 are surface proteins that facilitate interactions between T cell receptors for antigen and antigen itself which is presented to T cells by antigen-presenting cells. Antigens recognized by T cells are contained within clefts of major histocompatibility complex (MHC) proteins expressed on the surface of antigen-presenting cells. CD4 cells recognize peptide fragments presented by class II histocompatibility alleles, CD8 cells recognize fragments presented by class I histocompatibility alleles. The DR2 allele, over-represented in multiple sclerosis, is a MHC class II allele, pointing to a role for CD4⁺ cells in lesion formation in multiple sclerosis.

CD4⁺ cells can be further subdivided into Th1 and Th2 subtypes. Th1 cells are responsible for delayed type hypersensitivity responses and secrete numerous cytokines including interleukin-2 (IL-2), a stimulator of T cell proliferation, and interferon, an activator of macrophages, and lymphotoxin, a protein which has the capacity to damage oligodendrocytes, the myelin-forming cells of the CNS. Interferon administration provokes multiple sclerosis attacks, and elevated numbers of IF-secreting cells can be detected in the blood during attacks and in MS plaques. Interferon, in combination with IL-2 activates macrophages which directly strip myelin from nerve fibers as well as sectrete tumor necrosis factor-α (TNF-α), a cytokine that damages the myelin-producing oligodendrocytes.

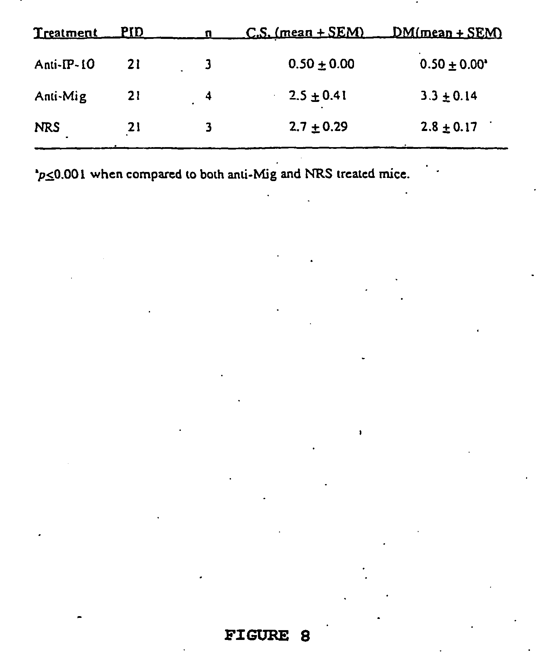

As described herein, neutralization of IP-10, which is expressed during the chronic stages of neurological disease almost exclusively in the area of demyelination, causes remyelination of demyelinated nerve fibers and reduces the further progression of demyelination (Figure 8). As such, neutralizing IP-10 activity can lead to a reduction in the severity of the demyelinating disease as well as to the level of neurological impairments associated with demyelination. IP-10 neutralizing agents including antibodies, antisense nucleic acids and compounds identified by the methods described below are useful for treating or reducing the severity of a demyelinating disease and for promoting remyelination in a subject affected with a demyelinating disease.

An IP-10 specific neutralizing agent modulates IP-10, sufficiently to reduce activity of IP-10 related to cell recruitment, demyelination and the prevention of remyelination. An IP-10 specific neutralizing agent can modulate the activity of IP-10 both through binding and non-binding interactions. An IP-10 specific neutralizing agent can be a macromolecule, such as polypeptide or nucleic acid. An IP-10 specific neutralizing agent can also be a derivative, analogue or mimetic compound as well as a small organic compound as long as IP-10 activity is reduced in the presence of the neutralizing agent. The size of a neutralizing agent is not important so long as the molecule exhibits or can be made to exhibit selective neutralizing activity towards IP-10. For example, a neutralizing agent can be as little as between about one and six, and as large as tens or hundreds of monomer building blocks which constitute a macromolecule or chemical binding molecule. Similarly, an organic compound can be a simple or complex structure so long as it binds IP-10 with sufficient affinity to reduce activity.

Neutralizing agents specific for IP-10 can include, for example, antibodies and other receptor or ligand binding polypeptides of the immune system. Such other molecules of the immune system include for example, chemokine receptors, as well as any other receptors or fragments thereof that bind IP-10, or can be made to bind IP-10, with sufficient affinity to reduce activity are also neutralizing agents useful for practicing the methods of the invention.

Various approaches can be used for identifying neutralizing agents selective for IP-10. For example, one approach is to use the information available regarding the structure and function of IP-10, or a modulator of IP-10, to binding molecule populations, from molecules known to function as chemokine binding molecules or known to exhibit or be capable of exhibiting binding affinity specific for IP-10, or a modulator of IP-10, such as fragments or mimetics of the CXCR3 receptor found on CD4+ T cells and NK cells. A neutralizing agent specific for IP-10 can be an antibody and other receptor of the immune repertoire. The normal function of such immune receptors is to bind essentially an infinite number of different antigens and ligands. Therefore, generating a diverse population of binding molecules from an immune repertoire, for example, can be useful for identifying a neutralizing agent specific for IP-10.

A neutralizing agent specific for IP-10 can further be identified from a large population of unknown molecules by methods well known in the art. Such a population can be a random library of peptides or small molecule compounds. The population can be generated to contain a sufficient diversity of sequence or structure so as to contain a molecule which will bind to the IP-10 protein or their respective nucleic acids. Those skilled in the art will know what size and diversity is necessary or sufficient for the intended purpose. A population of sufficient size and complexity can be generated so as to have a high probability of containing an IP-10 neutralizing agent that binds IP-10 with sufficient affinity to decrease activity. Numerous other types of library molecule populations exist and are described further below.

A neutralizing agent of the invention can modulate IP-10 expression or activity sufficiently to decrease their activity is useful for practicing the claimed methods of reducing the severity of a demyelinating disease and promoting remyelination in a subject affected with a demyelinating disease.

A moderate sized population for identification of an IP-10 specific neutralizing agent can consist of hundreds and thousands of different molecules, for example, binding molecules, within the population whereas a large population will consist of tens of thousands and millions of different molecule species. More specifically, large and diverse populations of molecules for the identification of a neutralizing agent will contain any of about 10⁴, 10⁵, 10⁶, 10⁷, 10⁸, 10⁹, 10¹⁰, or more, different molecule species. One skilled in the art will know the approximate diversity of the population of molecules sufficient to identify a neutralizing agent that modulates or regulates IP-10 activity or expression.

Recombinant libraries of molecules, for example, binding molecules, can be used to identify a neutralizing agent specific for IP-10 since large and diverse populations can be rapidly generated and screened with IP-10. Recombinant libraries of expressed polypeptide useful for identifying a neutralizing agent specific for IP-10 can be engineered in a large number of different ways known in the art. Recombinant library methods similarly allow for the production of a large number of binding molecule populations from naturally occurring repertoires. Whether recombinant or otherwise, essentially any source of binding molecule population can be used so long as the source provides a sufficient size and diversity of different binding molecules to identify a neutralizing agent specific for IP-10. If desired, a population of binding molecules useful for identifying a neutralizing agent specific for IP-10 can be a selectively immobilized to a solid support as described by Watkins et al., Anal. Biochem. 256 (92): 169-177 (1998).

A phage expression library in which lysogenic phage cause the release of bacterially expressed binding molecule polypeptides is a specific example of a recombinant library that can be used to identify a neutralizing agent specific for IP-10. In another type of phage expression library, large numbers of potential binding molecules can be expressed as fusion polypeptides on the periplasmic surface of bacterial cells. Libraries in yeast and higher eukaryotic cells exist as well and are similarly applicable in the methods of the invention. Those skilled in the art will know or can determine what type of library is useful for identifying a neutralizing agent specific for IP-10.

In addition to the methods described above, which utilize purified polypeptide to screen libraries of compounds for those which specifically bind IP-10, a neutralizing agent specific for IP-10 can be identified by using purified polypeptide to produce antibodies. For example, antibodies which are specific for IP-10 can be used as neutralizing agents of the invention and can be generated using methods that are well known in the art. Neutralizing agents useful for practicing the methods of the invention include both polyclonal and monoclonal antibodies against IP-10, or any molecule that modulates IP-10 expression or activity, as well as antigen binding fragments of such antibodies including Fab, F(ab')2, Fd and Fv fragments and the like. In addition, neutralizing agents useful for practicing the methods of the invention encompass non-naturally occurring antibodies, including, for example, single chain antibodies, chimeric antibodies, bifunctional antibodies, complementarity determining region-grafted (CDR-grafted) antibodies and humanized antibodies, as well as antigen-binding fragments thereof.

Methods of preparing and isolating antibodies, including polyclonal and monoclonal antibodies, using peptide immunogens, are well known to those skilled in the art and are described, for example, in Harlow and Lane, Antibodies: A Laboratory Manual, Cold Spring Harbor Laboratory Press (1988). Non-naturally occurring antibodies can be constructed using solid phase peptide synthesis, can be produced recombinantly or can be obtained, for example, by screening combinatorial libraries consisting of variable heavy chains and variable light chains as described by Huse et al., Science 246:1275-1281 (1989). These and other methods of making, for example, chimeric, humanized, CDR-grafted, single chain, and bifunctional antibodies are well known to those skilled in the art (Hoogenboom et al., U.S. Patent No. 5,564,332, issued October 15, 1996; Winter and Harris, Immunol. Today 14:243-246 (1993); Ward et al., Nature 341:544-546 (1989) ; Harlow and Lane, Antibodies: A laboratory manual (Cold Spring Harbor Laboratory Press, 1988); Hilyard et al., Protein Engineering: A practical approach (IRL Press 1992); Borrabeck, Antibody Engineering, 2d ed. (Oxford University Press 1995)).

An IP-10 specific antibody neutralizing agent can be raised using as an immunogen a substantially purified IP-10 protein, which can be prepared from natural sources or produced recombinantly, or a peptide portion of an IP-10 protein including synthetic peptides. A non-immunogenic peptide portion of a IP-10 protein can be made immunogenic by coupling the hapten to a carrier molecule such bovine serum albumin (BSA) or keyhole limpet hemocyanin (KLH), or by expressing the peptide portion as a fusion protein. Various other carrier molecules and methods for coupling a hapten to a carrier molecule are well known in the art (see Harlow and Lane, supra, 1988; see, also, Hermanson, Bioconjugate Techniques, Academic Press, 1996). As described above, an antibody neutralizing agent specific for IP-10 can also be raised against a regulatory molecule that modulates IP-10 expression or activity rather than against IP-10 directly.

A neutralizing agent specific for IP-10, such as an antibody can be labeled so as to be detectable using methods well known in the art (Hermanson, supra, 1996; Harlow and Lane, supra, 1988; chap. 9). For example, a neutralizing agent specific for IP-10 can be linked to a radioisotope or therapeutic agent by methods well known in the art. A neutralizing agent that directly binds IP-10 linked to a radioisotope or other moiety capable of visualization can be useful to diagnose or stage the progression of a clinical stage of a demyelinating disease or neuroinflammatory disease that is characterized by the organ or tissue-specific presence or absence of IP-10. Thus, the invention also provides diagnostic methods applicable to conditions that are associated with IP-10 activity or expression.

Methods for raising polyclonal antibodies, for example, in a rabbit, goat, mouse or other mammal, are well known in the art (Harlow and Lane, supra, 1988). The production of anti-peptide antibodies commonly involves the use of host animals such as rabbits, mice, guinea pigs, or rats. If a large amount of serum is needed, larger animals such as sheep, goats, horses, pigs, or donkeys can be used. Animals are usually chosen based on the amount of antiserum required and suitable animals include rabbits, mice, rats, guinea pigs, and hamsters. These animals yield a maximum of 25 mL, 100-200 µl, 1-2 mL, 1-2 mL, and 1-2 mL of serum per single bleed, respectively (Harlow and Lane, supra, 1988). Rabbits are very useful for the production of polyclonal antisera, since they can be safely and repeatedly bled and produce high volumes of antiserum. Two injections two to four weeks apart with 15-50µg of antigen in a suitable adjuvant such as, for example, Freund's Complete Adjuvant can be followed by blood collection and analysis of the antiserum.

In addition, monoclonal antibodies can be obtained using methods that are well known and routine in the art (Harlow and Lane, supra, 1988). A peptide portion of a protein such as IP-10 for use as an immunogen can be determined by methods well known in the art. Spleen cells from an IP-10 immunized mouse can be fused to an appropriate myeloma cell line to produce hybridoma cells. Cloned hybridoma cell lines can be screened using a labeled IP-10 protein to identify clones that secrete anti-IP-10 antibodies, respectively. Hybridomas expressing anti-IP-10 monoclonal antibodies having a desirable specificity and affinity can be isolated and utilized as a continuous source of the antibody neutralizing agent.

Neutralizing agents specific for IP-10 can be used to reduce the severity of a demyelinating disease in a mammal. Neutralizing agents specific for IP-10 can promote remyelination in a subject affected with a demyelinating disease in a mammal. Thus, neutralizing agents specific for IP-10 can be used to reduce the severity of a demyelinating disease in a human subject. Furthermore, neutralizing agents specific for IP-10 can be used to promote remyelination in a subject affected with a demyelinating disease in a human subject. Humanized antibodies can be constructed by conferring essentially any antigen binding specificity onto a human antibody framework. Methods of constructing humanized antibodies are useful to prepare an antibody neutralizing agent appropriate for practicing the methods of the invention and avoiding host immune responses against the antibody neutralizing agent when used therapeutically.

The antibody neutralizing agents described above can be used to generate therapeutic human neutralizing agents by methods well known in the art such as complementary determining region (CDR)-grafting and optimization of framework and CDR residue. For example, humanization of an antibody neutralizing agent can be accomplished by CDR-grafting as described in Fiorentini at al., Immunotechnology 3(1): 45-59 (1997). Briefly, CDR-grafting involves recombinantly splicing CDRs from a nonhuman antibody neutralizing agent into a human framework region to confer binding activity onto the resultant grafted antibody, or variable region binding fragment thereof. Once the CDR-grafted antibody, or variable region binding fragment is made, binding affinity comparable to the nonhuman antibody neutralizing agent can be reacquired by subsequent rounds of affinity maturation strategies known in the art. Humanization of antibody neutralizing agents in the form of rabbit polyclonal antibodies can be accomplished by similar methods as described in Rader et al., J. Biol. Chem. 275(18): 13668-13676 (2000).

Humanization of a nonhuman IP-10 antibody neutralizing agent can also be achieved by simultaneous optimization of framework and CDR residues, which permits the rapid identification of co-operatively interacting framework and CDR residues, as described in Wu et al., J. Mol. Biol. 294 (1): 151-162 (1999). Briefly, a combinatorial library that examines a number of potentially important framework positions is expressed concomitantly with focused CDR libraries consisting of variants containing random single amino acid mutations in the third CDR of the heavy and light chains. By this method, multiple Fab variants containing as few as one nonhuman framework residue and displaying up to approximately 500-fold higher affinity than the initial chimeric Fab can be identified. Screening of combinatorial framework-CDR libraries permits identification of monoclonal antibodies with structures optimized for function, including instances in which the antigen induces conformational changes in the monoclonal antibody. The enhanced humanized variants contain fewer nonhuman framework residues than antibodies humanized by sequential in vitro humanization and affinity maturation strategies known in the art.

It is further contemplated that a neutralizing agent of the invention can be a human antibody or a primatized antibody. In addition, human antibodies can be produced by methods known in the art that involve immunizing a transgenic nonhuman animal with the desired antigen. The transgenic nonhuman animal can be modified such that it fails to produce endogenous antibodies, but instead produces B-cells which secrete fully human immunoglobulins. The antibodies produced can be obtained from the animal directly or from immortalized B-cells derived from the transgenic nonhuman animal. Alternatively, the genes encoding the immunoglobulins with human variable regions can be recovered and expressed to obtain the antibodies directly or modified to obtain analogs of antibodies such as, for example, single chain Fᵥ molecules. Thus, it is contemplated to produce a neutralizing agent of the invention that is a fully human immunoglobulin to a specific antigen or to produce an analog of the immunoglobulin by a process that includes immunizing a nonhuman animal with antigen under conditions that stimulate an immune response.

The nonhuman animal that produces a human antibody neutralizing agent of the invention can be modified to be substantially incapable of producing endogenous heavy or light immunoglobulin chain, but capable of producing immunoglobulins with both human variable and constant regions. In the resulting immune response, the animal produces B cells which secrete immunoglobulins that are fully human and specific for the antigen. The human immunoglobulin of desired specificity can be directly recovered from the animal, for example, from the serum, or primary B cells can be obtained from the animal and immortalized. The immortalized B cells can be used directly as the source of human antibodies or, alternatively, the genes encoding the antibodies can be prepared from the immortalized B cells or from primary B cells of the blood or lymphoid tissue, for example, spleen, tonsils, lymph nodes, bone marrow, of the immunized animal and expressed in recombinant hosts, with or without modifications, to produce the neutralizing agent immunoglobulin or its analogs. In addition, the genes encoding the repertoire of immunoglobulins produced by the immunized animal can be used to generate a library of immunoglobulins to permit screening for those variable regions which provide the desired affinity. Clones from the library which have the desired characteristics can then be used as a source of nucleotide sequences encoding the desired variable regions for further manipulation to generate human antibodies or analogs with these characteristics using standard recombinant techniques. Various techniques for preparing human antibodies using transgenic nonhuman animals, for example, transgenic mice, are well known in the art and described, for example, in Fishwild et al., Nature Biotechnology 14: 845-851 (1996); Heijnen et al., Journal of Clinical Investigation 97: 331-338 (1996); Lonberg et al. Nature 368: 856-859 (1994); Morrison, Nature 368: 812-813 (1994); Neuberger, Nature Biotechnology 14: 826 (1996); Chadd and Chamow, Curr. Opin. Biotechnol. 12(2):188-94 (2001); Russel et al., Infection and Immunity 1820-1826 (2000); Gallo et al., European Journal of Immunology 30:534-540 (2000); Davis et al., Cancer Metastasis Rev. 18(4):421-5 (1999); Green, Journal of Immunological Methods 231:11-23 (1999); Yang et al., Journal of Leukocyte Biology 66:401-410 (1999); Jakobovits, Advanced Drug Delivery Reviews 31:33-42 (1998); Green and Jakobovits, J. Exp. Med. 188(3):483-495 (1998); Jakobovits, Exp. Opin. Invest. Drugs 7(4):607-614 (1998); Mendez et al., Nature Genetics 15:146-156 (1997); Jakobovits, Weir's Handbook of Experimental Immunology, The Integrated Immune System, Vol. IV: 194.1-194.7 (1996). Furthermore, various techniques known in the art for preparation of a human antibody are described in United States Patent Nos. 6,162,963; 6,150,584; 6,114,598; 6,111,166; 6,096,311 and 6,075,181.

Another highly efficient means for generating recombinant antibodies is by primatization as described by Newman, Biotechnology 10:1455-1460 (1992). More particularly, primatized antibodies can be generated which contain monkey variable domains and human constant sequences. Methods for primatization are known in the art and described in United States Patent No.6,113,898. Antibodies can be primatized such that they are not antigenically rejected upon human administration. Primatization relies on immunization of primates, for example, cynomolgus monkeys (*Macaca fascicularis*), with human antigens or receptors and can be used to create high affinity monoclonal antibodies directed to human cell surface antigens. Antibodies generated by primatization have previously been reported to display human effector function, have reduced immunogenicity, and long serum half-life and are thus useful as neutralizing agents of the invention. The technology relies on the fact that despite the fact that cynomolgus monkeys are phylogenetically similar to humans, they still recognize many human proteins as foreign and therefore mount an immune response. Moreover, because the cynomolgus monkeys are phylogenetically close to humans, the antibodies generated in these monkeys have been discovered to have a high degree of amino acid homology to those produced in humans. Indeed, after sequencing macaque immunoglobulin light and heavy chain variable region genes, it was found that the sequence of each gene family was 85-98% homologous to its human counterpart (Newman et al, supra, 1992). The first antibody generated by primatization, an anti-CD4 antibody, was 91-92% homologous to the consensus sequence of human immunoglobulin framework regions (Newman et al, supra, 1992). Methods known in the art for generation of a primatized antibody and useful for preparing a neutralizing agent of the invention further are described by Newman et al., Clinical Immunology 98 (2) :164-74 (2001) ; and Reddy et al., J Immunol. 164 (4) :1925-33 (2000).

As described above, antibody neutralizing agents of the invention include, for example, polyclonal antibodies, monoclonal antibodies as well as recombinant versions and functional fragments thereof. Recombinant versions of antibody neutralizing agents include a wide variety of constructions ranging from simple expression and co-assembly of encoding heavy and light chain cDNAs to speciality constructs termed designer antibodies. Recombinant methodologies, combined with the extensive characterization of polypeptides within the immunoglobulin superfamily, and particularly antibodies, provides the ability to design and construct a vast number of different types, styles and specificities of binding molecules derived from immunoglobulin variable and constant region binding domains. Specific examples include chimeric antibodies, where the constant region of one antibody is substituted with that of another antibody, and humanized antibodies, described above, where the complementarity determining regions (CDR) from one antibody are substituted with those from another antibody.

Other recombinant versions of antibody neutralizing agents include, for example, functional antibody variants where the variable region binding domain or functional fragments responsible for maintaining antigen binding is fused to an F_{c} receptor binding domain from the antibody constant region. Such variants are essentially truncated forms of antibodies that remove regions non-essential for antigen and F_{c} receptor binding. Truncated variants can be have single valency, for example, or alternatively be constructed with multiple valencies depending on the application and need of the user. Additionally, linkers or spacers can be inserted between the antigen and F_{c} receptor binding domains to optimize binding activity as well as contain additional functional domains fused or attached to effect biological functions other than IP-10 neutralization. Those skilled in the art will know how to construct recombinant antibody neutralizing agents specific for IP-10 in light of the art knowledge regarding antibody engineering and given the guidance and teachings herein. A description of recombinant antibodies, functional fragments and variants and antibody-like molecules can be found, for example, in "Antibody Engineering," 2nd Edition, (Carl A.K. Borrebaeck, Ed.) Oxford University Press, New York, (1995).

Additional functional variants of antibodies that can be used as antibody neutralizing agents include antibody-like molecules other than antigen binding-F_{c} receptor binding domain fusions. For example, antibodies, functional fragments and fusions thereof containing a F_{c} receptor binding domain can be produced to be bispecific in that one variable region binding domain exhibits binding activity for one antigen and the other variable region binding domain exhibits binding activity for a second antigen. Such bispecific antibody neutralizing agents can be advantageous in the methods of the invention because a single bispecific antibody will contain two different target antigen binding species.

An antibody neutralizing agent specific for IP-10 can also be an immunoadhesion or bispecific immunoadhesion. Immunoadhesions are antibody-like molecules that combine the binding domain of a non-antibody polypeptide with the effector functions of an antibody of an antibody constant domain. The binding domain of the non-antibody polypeptide can be, for example, a ligand or a cell surface receptor having ligand binding activity. Immunoadhesions for use as IP-10 neutralizing agents can contain at least the F_{c} receptor binding effector functions of the antibody constant domain. Specific examples of ligands and cell surface receptors that can be used for the antigen binding domain of an immunoadhesion neutralizing agent include, for example, a T cell or NK cell receptor such as the CXCR3 receptor that recognizes IP-10. Other ligands and ligand receptors known in the art can similarly be used for the antigen binding domain of an immunoadhesion neutralizing agent specific for IP-10. In addition, multivalent and multispecific immunoadhesions can be constructed for use as IP-10 and Mig neutralizing agents. The construction of bispecific antibodies, immunoadhesions, bispecific immunoadhesions and other heteromultimeric polypeptides which can be used as IP-10 specific neutralizing agents is the subject matter of, for example, U.S. Patent Numbers 5,807,706 and 5,428,130.

In one embodiment of the invention, the polynucleotides encoding IP-10, regulatory molecules that modulate the expression or activity of IP-10, or any fragment thereof, or antisense molecules, can be used as neutralizing agents for therapeutic purposes. In one aspect, antisense molecules to the IP-10 encoding nucleic acids can be used to block the transcription or translation of the mRNA. Specifically, cells can be transformed with sequences complementary to IP-10 nucleic acids. Such methods are well known in the art, and sense or antisense oligonucleotides or larger fragments, can be designed from various locations along the coding or control regions of sequences encoding IP-10. Thus, antisense molecules may be used to neutralize IP-10 activity, or to achieve regulation of gene function.

The activity of IP-10 as well as the activity of a neutralizing agent can be confirmed by in vitro assays known in the art. Thus, the invention further relates to a method of identifying a neutralizing agent specific for IP-10 comprising contacting cells containing an IP-10 receptor with IP-10 in the presence of a candidate neutralizing agent, and determining the occurrence of a biological response associate with IP-10 activity, wherein non-occurrence of the biological response identifies the candidate as an IP-10 neutralizing agent.

As described in Example III, a chemotaxis assay can be used to evaluate the ability of defined signals to induce directional migration of a targeted cell population. Briefly, the activity of IP-10 can be neutralized with a neutralizing agent of the invention, for example, a human monoclonal antibody and the inhibitory effect of this treatment on T cell migration into the central nervous system can be confirmed. As further described in Example III, an assay that measures intracellular calcium changes also is useful to confirm the activity of a neutralizing agent of the invention. In this regard, a hallmark of chemokine binding to a specific chemokine receptor is a change in intracellular calcium levels. Therefore, the ability of a neutralizing agent to inhibit the interaction of IP-10 with the CXCR3 receptor can be confirmed by measuring and comparing intracellular calcium changes in the absence versus the presence of a neutralizing agent of the invention. Other assays for identifying a neutralizing agent specific for IP-10 that are known in the art aslo can be used to practice the invention.

Expression vectors derived from retroviruses, adenovirus, adeno-associated virus (AAV), herpes or vaccinia viruses, or from various bacterial plasmids can be used for delivery of antisense nucleotide sequences. The viral vector selected should be able to infect the central nervous system cells and be safe to the host and cause minimal cell transformation. Retroviral vectors and adenoviruses offer an efficient, useful, and presently the best-characterized means of introducing and expressing foreign nucleotide sequences efficiently in mammalian cells. These vectors are well known in the art and have very broad host and cell type ranges, express genes stably and efficiently. Methods well known to those skilled in the art can be used to construct such recombinant vectors and are described in Sambrook et al., Molecular Clowning: A Laboratory Manual, 2nd ed., Cold Spring Harbor Press, Plainview, New York (1989), and Ausubel et al., Current Protocols in Molecular Biology, John Wiley & Sons, New York (1999). Even in the absence of integration into the DNA, such vectors can continue to transcribe RNA molecules until they are disabled by endogenous nucleases. Transient expression can last for a month or more with a non-replicating vector and even longer if appropriate replication elements are part of the vector system.

Ribozymes, enzymatic RNA molecules, can also be used to catalyze the specific cleavage of IP-10 mRNA, or the mRNA of any regulatory molecule that modulates the expression or activity of IP-10. The mechanism of ribozyme action involves sequence-specific hybridization of the ribozyme molecule to complementary target the mRNA, followed by endonucleolytic cleavage. Specific ribozyme cleavage sites within any potential RNA target are identified by scanning the RNA for ribozyme cleavage sites which include the following sequences: GUA, GUU, and GUC. Once identified, short RNA sequences of between 15 and 20 ribonucleotides corresponding to the region of the target gene containing the cleavage site can be evaluated for secondary structural features which can render the oligonucleotide inoperable. The suitability of candidate targets can also be evaluated by testing accessibility to hybridization with complementary oligonucleotides using ribonuclease protection assays. Antisense molecules and ribozymes of the invention can be prepared by any method known in the art for the synthesis of nucleic acid molecules.

The neutralizing agents useful for practicing the methods of the invention can be formulated and administered by those skilled in the art in a manner and in an amount appropriate for the demyelinating disease to be treated; the rate or amount of demyelination; the weight, gender, age and health of the subject; the biochemical nature, bioactivity, bioavailability and side effects of the particular compound; and in a manner compatible with concurrent treatment regimens. An appropriate amount and formulation for decreasing the severity of a demyelinating disease in humans can be extrapolated from credible animal models known in the art of the particular disorder. It is understood, that the dosage of a neutralizing agent specific for IP-10 has to be adjusted based on the binding affinity of the neutralizing agent for IP-10, such that a lower dose of a neutralizing agent exhibiting significantly higher binding affinity can be administered compared to the dosage necessary for a neutralizing agent with lower binding affinity.

The total amount of neutralizing agent can be administered as a single dose or by infusion over a relatively short period of time, or can be administered in multiple doses administered over a more prolonged period of time. Such considerations will depend on a variety of case-specific factors such as, for example, whether the disease category is characterized by acute episodes or gradual neurologic deterioration. For example, for a subject affected with chronic neurologic deterioration the neutralizing agent can be administered in a slow-release matrice, which can be implanted for systemic delivery or at the site of the target tissue. Contemplated matrices useful for controlled release of therapeutic compounds are well known in the art, and include materials such as DepoFoamTM, biopolymers, micropumps, and the like.

The neutralizing agents of the invention can be administered to the subject by any number of routes known in the art including, for example, systemically, such as intravenously or intraarterially. An IP-10 specific neutralizing agent can be provided in the form of isolated and substantially purified polypetides and polypeptide fragments in pharmaceutically acceptable formulations using formulation methods known to those of ordinary skill in the art. These formulations can be administered by standard routes, including for example, topical, transdermal, intraperitoneal, intracranial, intracerebroventricular, intracerebral, intravaginal, intrauterine, oral, rectal or parenteral (e.g., intravenous, intraspinal, intrathecal, subcutaneous or intramuscular) routes. Intrathecal administration of an IP-10 specific neutralizing agent into the intradural or subarachnoid space is a preferred route for practicing the methods of the invention. Intravenous administration of an IP-10 specific neutralizing agent also is a preferred route for practicing the methods of the invention. In addition, an IP-10 specific neutralizing agent variant can be incorporated into biodegradable polymers allowing for sustained release of the compound useful for reducing the severity of a demyelinating disease. Biodegradable polymers and their use are described, for example, in Brem et al., J. Neurosurg, 74:441-446 (1991).

An IP-10 specific neutralizing agent can be administered as a solution or suspension together with a pharmaceutically acceptable medium. Such a pharmaceutically acceptable medium can be, for example, sterile aqueous solvents such as sodium phosphate buffer, phosphate buffered saline, normal saline or Ringer's solution or other physiologically buffered saline, or other solvent or vehicle such as a glycol, glycerol, an oil such as olive oil or an injectable organic ester. A pharmaceutically acceptable medium can additionally contain physiologically acceptable compounds that act, for example, stabilize the neutralizing agent, increase its solubility, or increase its absorption. Such physiologically acceptable compounds include, for example, carbohydrates such as glucose, sucrose or dextrans; antioxidants such as ascorbic acid or glutathione; receptor mediated permeabilizers, which can be used to increase permeability of the blood-brain barrier; chelating agents such as EDTA, which disrupts microbial membranes; divalent metal ions such as calcium or magnesium; low molecular weight proteins; lipids or liposomes; or other stabilizers or excipients. Those skilled in the art understand that the choice of a pharmaceutically acceptable carrier depends on the route of administration of the compound containing the neutralizing agent and on its particular physical and chemical characteristics.

Formulations suitable for parenteral administration include aqueous and non-aqueous sterile injection solutions such as the pharmaceutically acceptable mediums described above. The solutions can additionally contain, for example, buffers, bacteriostats and solutes which render the formulation isotonic with the blood of the intended recipient. Other formulations include, for example, aqueous and non-aqueous sterile suspensions which can include suspending agents and thickening agents. The formulations can be presented in unit-dose or multi-dose containers, for example, sealed ampules and vials, and can be stored in a lyophilized condition requiring, for example, the addition of the sterile liquid carrier, immediately prior to use. Extemporaneous injection solutions and suspensions can be prepared from sterile powders, granules and tablets of the kind previously described.

For applications that require the compounds and compositions to cross the blood-brain barrier, formulations that increase the lipophilicity of the compound are particularly desirable. For example, the neutralizing agent can be incorporated into liposomes (Gregoriadis, Liposome Technology, Vols. I to III, 2nd ed. (CRC Press, Boca Raton FL (1993)). Liposomes, which consist of phospholipids or other lipids, are nontoxic, physiologically acceptable and metabolizable carriers that are relatively simple to make and administer.

A neutralizing agent specific for IP-10 can also be prepared as nanoparticles. Adsorbing peptide compounds onto the surface of nanoparticles has proven effective in delivering peptide drugs to the brain (see Kreuter et al., Brain Res. 674:171-174 (1995)). Exemplary nanoparticles are colloidal polymer particles of poly-butylcyanoacrylate with a neutralizing agent specific for IP-10 adsorbed onto the surface and then coated with polysorbate 80.

Image-guided ultrasound delivery of a neutralizing agent through the blood-brain barrier to selected locations in the brain can be utilized as described in U.S. Patent No. 5,752,5115. Briefly, to deliver a neutralizing agent past the blood-brain barrier a selected location in the brain is targeted and ultrasound used to induce a change detectable by imaging in the central nervous system (CNS) tissues and/or fluids at that location. At least a portion of the brain in the vicinity of the selected location is imaged, for example, via magnetic resonance imaging (MRI), to confirm the location of the change. An IP-10 specific neutralizing in the patient's bloodstream can delivered to the confirmed location by applying ultrasound to effect opening of the blood-brain barrier at that location and, thereby, to induce uptake of the neutralizing agent.

In addition, polypeptides called receptor mediated permeabilizers (RMP) can be used to increase the permeability of the blood-brain barrier to molecules such as therapeutic agents or diagnostic agents as described in U.S. Patent Nos. 5,268,164; 5,506,206; and 5,686,416. These receptor mediated permeabilizers can be intravenously co-administered to a host with molecules whose desired destination is the cerebrospinal fluid compartment of the brain. The permeabilizer polypeptides or conformational analogues thereof allow therapeutic agents to penetrate the blood-brain barrier and arrive at their target destination.

In current treatment regimes for demyelinating diseases, more than one compound is often administered to an individual for management of the same or different aspects of the disease. Similarly, in the methods of the invention involving decreasing the rate of or reversing demyelination, a neutralizing agent specific for IP-10 can advantageously be formulated with a second therapeutic compound such as an anti-inflammatory compound, immunosuppressive compound or any other compound that manages the same or different aspects of the disease. Such compounds include, for example, methylprednisolone acetate, dexamethasone and betamethasone. Contemplated methods of reducing the severity of a demyelinating disease and promoting remyelination include administering a neutralizing agent specific for IP-10 alone, in combination with, or in sequence with, such other compounds. Alternatively, combination therapies can consist of fusion proteins, where the neutralizing agent specific for IP-10 is linked to a heterologous protein, such as a therapeutic Protein.

The following examples are intended to illustrate but not limit the present invention.

### EXAMPLE I

### Selective Inhibition of IP-10 with Anti-IP-10 Antibody

This example describes selective inhibition of IP-10 in mice with a rabbit polyclonal anti-IP-10 antibody, which resulted in increased mortality and decreased T lymphocyte infiltration of the CNS in response to viral infection. In addition, the selective inhibition of IP-10 in mice following viral infection of the CNS resulted in a reduction in demyelination of nerve fibers that was accompanied by a gross increase in remyelination.

In order to determine the functional significance of IP-10 expression following viral infection of the CNS, mice were infected with a neurotropic strain of MHV, a positive-strand RNA virus. Intracranial infection of susceptible mice with MHV results in a chronic demyelinating disease that shares many clinical and histologic similarities with MS. As such, the MHV mouse model is a well accepted animal model for studying the underlying immunopathological mechanisms contributing to the pathogenesis of MS. Specifically, intracranial infection of mice with MHV results in an acute encephalomyelitis followed by chronic neurological disease in susceptible strains of mice (see Houtman and Fleming, J. Neurovirol. 2:361-376(1996); Buchmeier and Lane, Curr. Opin. Micro. 2:398-402 (1999)). The acute stage of MHV is represented by widespread viral infection of neurons and glial cells, whereas the chronic stage is characterized by viral persistence in astrocytes and oligodendrocytes accompanied by mononuclear cell infiltration and myelin destruction (see Houtman and Fleming, supra, 1996; Buchmeier and Lane, supra, 1999).

The Mouse Hepatitis Virus (MHV) strain V5A13.1 was derived from wild-type MHV-4 as described by Dalziel et al., J. Virol. 59: 463-471 (1986). Age matched five to seven week old male wt C57BL/6 (H-2b background) mice (Sprague-Dawley, San Diego, CA) were used for the described studies. Following anesthetization by inhalation of methoxyfluorane (Pitman-Moore Inc., Washington Crossing, NJ), the mice were injected intracranially with 10pfu of MHV suspended in 30µl of sterile saline as described in Lane et al., J. Virol. 74:1415-1424 (2000).

Control animals were injected with sterile saline alone. The animals were sacrificed at days 7 and 10 post infection, at which time brains and spinal cords were removed. One-half of each brain was used for the plaque assay on the DBT astrocytoma cell line to determine viral burden as described in Lane et al., supra, 2000. The remaining half of each brain was either fixed for histological analysis, stored at -80°C for RNA isolation, or used for FACS analysis or ELISA. All data were analyzed by performing the Mann-Whitney Rank Sum test using Sigma Stat 2.0 software, with P values of 0.05 or smaller considered significant.

IP-10 is expressed very early (day 1 post infection) within the CNS following MHV infection and remains the predominant chemokine expressed during the acute phase of the disease (see Lane et al., J. Immunol. 160:970-978 (1998)). IP-10 activity was selectively inhibited through intraperitoneal administration of rabbit polyclonal anti-IP-10 antisera.

The rabbit polyclonal antisera specific for mouse IP-10 was generated as described in Tannenbaum et al., J. Immunol. 161: 927-932 (1998). The rabbit polyclonal antibody has been shown previously to be specific for IP-10 and not cross-react with other known chemokines (see Tannenbaum et al., supra, 1998). MHV-infected mice were divided into two groups and treated with either normal rabbit serum (NRS) or anti-IP-10. Mice were injected intraperitoneally with 0.5 ml of anti-IP-10 antisera at a concentration of 0.5 mg/ml or with NRS on days 0, 2, 5, 7, and 9 post infection and subsequently sacrificed at days 7 and 10 post infection.

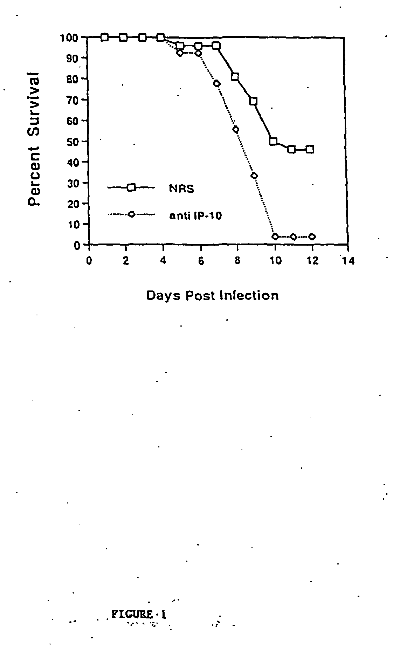

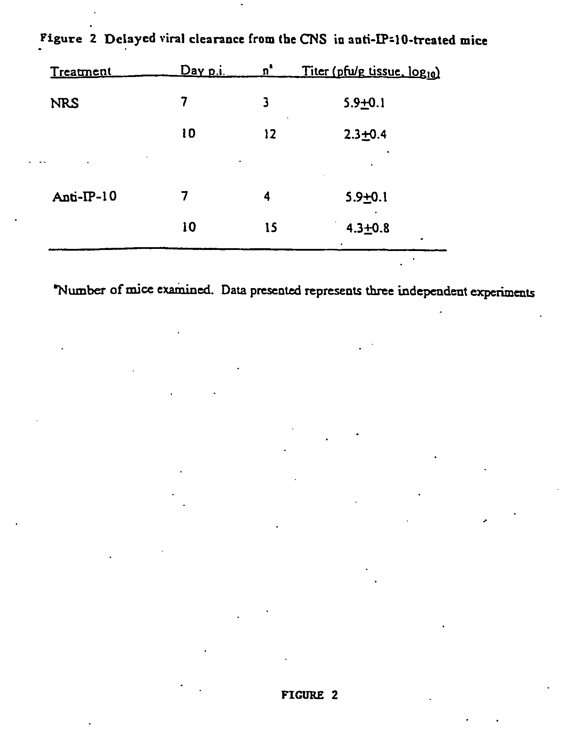

Treatment with rabbit polyclonal antiserum specific for mouse IP-10 led to an increase in mortality with less than 5 percent of any anti-IP-10 mice surviving until day 12 post infection (n=27)(Figure 1). In contrast, approximately 50 percent of NRS treated control mice survived MHV infection(n=27) (Figure 1). Correlating with increased mortality was a decrease in the ability of anti-IP-10-treated mice to clear virus from the CNS as compared to NRS-treated mice. Specifically, surviving anti-IP-10 treated mice displayed a 2-log increase in viral titers in the brain as compared to titers present in NRS-treated mice at day 10 post infection (Figure 2).

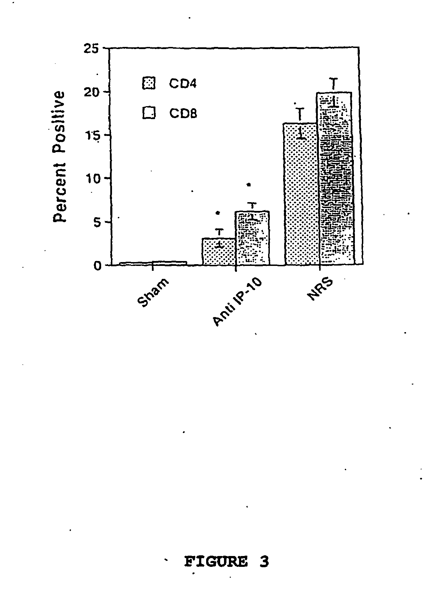

Previous studies have shown that CD4⁺ and CD8⁺ cells are important in clearing MHV from the CNS (see Williamson and Stohlman, J. Virol. 64:4589-4592 (1990); Yamaguchi et al., J. Neuroimmunol. 32:1-9 (1991); Pearce et al., J. Virol. 68:5483-5495 (1997); Lane et al., supra, 2000). In this study, cells were obtained from brains of anti-IP-10 or NRS treated MHV-infected mice at 7 days post infection and dual fluorescent staining was performed for CXCR3 and CD4 or CD8 antigen as preciously described in Lane et al., supra, 2000. Fluorescein isothiocyanate-conjugated (FITC) rat anti-mouse antibody was used to detect CD4 and CD8 positive T cells (Pharmingen, San Diego, CA). As a control, an isotype-matched FITC-conjugated antibody was used. Cells were incubated with antibodies for 30 minutes at 4°C, washed and subsequently fixed in 1 percent paraformaldehyde and finally analyzed on a FACStar (Becton Dickinson, Mountain View, CA). Dual fluorescent staining CD4⁺ and CD8⁺ lymphocytes as well as confocal microscopy showed that the majority of CD4⁺ and CD8⁺ lymphocytes that infiltrate the CNS in response to viral infection express the IP-10 receptor CXCR3. CD4⁺ and CD8⁺ T lymphocytes expressing CXCR3 were present within the meninges as well as the parenchyma indicating these cells were able to migrate into the brain. Overall, these results demonstrate that IP-10 is a modulator of the host defense by attracting T cells into the CNS in response to viral infection. Furthermore, as shown in Figure 3, flow-cytometric analysis revealed that anti-IP-10 treatment of mice resulted in a 82.3 percent decrease in infiltrating CD4+ T cells and a 70.4 percent decrease in infiltrating CD8+ cells compared to NRS treatment of mice. Both, anti-IP-10 treated and NRS treated mice displayed comparable levels of monocyte/macrophage infiltration, indicating that IP-10 does not attract these cells into the CNS following viral infection.

The number of T lymphocytes infiltrating the CNS in anti-IP-10 treated mice and the CXCR3 expression on infiltrating CD4⁺ and CD8⁺ T lymphocytes was determined with primary antibodies diluted in buffered saline containing 5 percent normal horse serum used for dual fluorescent detection of cellular antigens as follows: Rat anti-mouse CD4 (Pharmingen, San Diego, CA) at a dilution of 1:100; and goat anti-mouse CXCR3 (Santa Cruz Laboratories, Santa Cruz, CA) at a dilution of 1:50. For CD4 and CD8 primary antibodies, a TRITC-conjugated secondary antibody was used at a dilution of 1:50 (Sigma, ST. Louis, MO). For CXCR3 primary antibody, an FITC conjugated secondary antibody was used at a dilution of 1:50 (Zymed, South San Francisco, CA). The reagent used to detect F4/80, an antigen specific to macrophages, is a monoclonal antibody (clone C1:A3-1) (Serotec, Raleigh, NC) which was used at a 1:50 dilution in a 2% goat serum/PBS solution according to the manufacturer's instructions. Staining was performed on 8µm frozen sections fixed in acetone for 10 minutes at -20°C. Subsequently, dual stained slides were subjected to confocal microscopy using a BioRad MRC UV laser scanning confocal microscope.

To determine whether infiltrating T lymphocytes contribute to the host defense against MHV infection of the CNS through the release of the anti-viral cytokine IFN-γ, levels of mRNA and protein within the brains of anti-IP-10 and NRS treated mice were determined by ribonuclease protection assay (RPA) and ELISA, respectively.

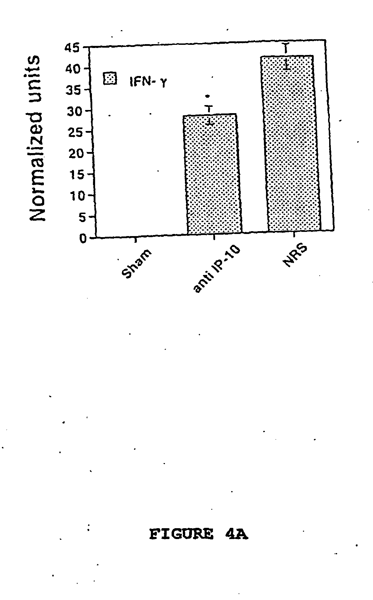

Total RNA was extracted from the brains of NRS treated or anti-IP-10 treated mice at day 7 post infection using TRIzol® (Life Technologies (GIBCO-BRL) Rockville, MD) reagent as described in Lane et al., supra, 2000; and Lane et al., J. Immunol., 160:970-978 (1998). The ribonuclease protection assay was performed using 15 µg of total RNA as previously described in Lane et al., supra, 2000; and Lane et al., supra, 1998. Interferon-γ (IFN-γ) levels were quantified using the Quantikine M mouse IFN-γ immunoassay kit (R&D Systems, Minneapolis, MN) using brains of mice obtained at day 7 post infection as described in Lane et al., supra, 2000. The data shown in Figure 4A is presented as normalized units representing the ratio of signal intensity of IFN-γ to internal L32 included in the probe set. Analysis of the values obtained from the scanned autoradiograph was performed using NIH Image 1.61 software.

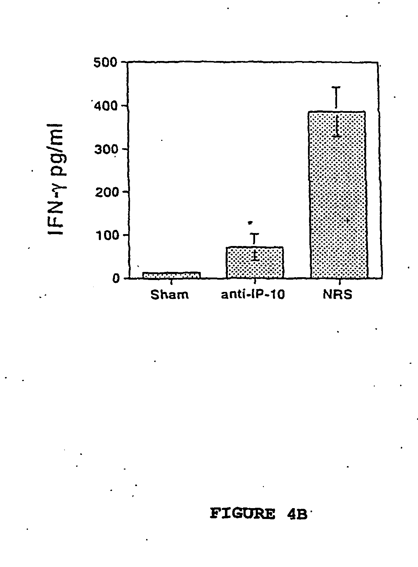

As shown in Figure 4A, the neutralization of IP-10 resulted in decreased mRNA transcripts for IFN-γ as compared to transcript levels present in the brains of NRS-treated mice. Correlating with the decrease in IFN-γ transcript levels was an approximate 80 percent decrease in IFN-γ protein levels at day 7 post infection compared to protein levels in NRS treated mice (Figure 4B). Although the levels of IFN-γ mRNA transcripts in anti-IP10 treated mice were slightly higher than would be predicted based on the IFN-γ ELISA data, this is most likely due to mouse-to-mouse variation and sensitivity in the RPA and not the result of IP-10 modulating IFN-γ mRNA translation.

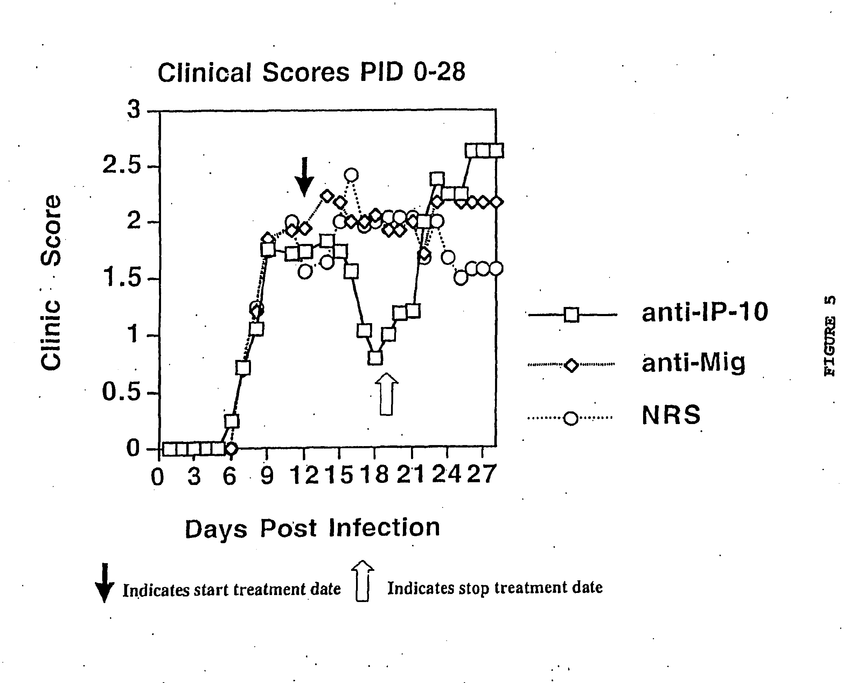

To determine the effect of a continuous course of anti-IP-10 and anti-Mig treatment on MHV infected mice, treatment was administered continuously from 12 days until 19 day's post infection. Figure 5 shows the clinical score of MHV infected mice treated with either anti-IP-10, anti-Mig or NRS starting at 12 days and ending at 19 days post infection. The clinical score for the three populations was substantially identical until the start of treatment and remained substantially identical for mice treated with anti-Mig and those treated with NRS (Figure 5). However, the clinical score for anti-IP-10 treated mice significantly declined compared to the anti-Mig and NRS treated mice during treatment and recovered once treatment was discontinued (Figure 5).

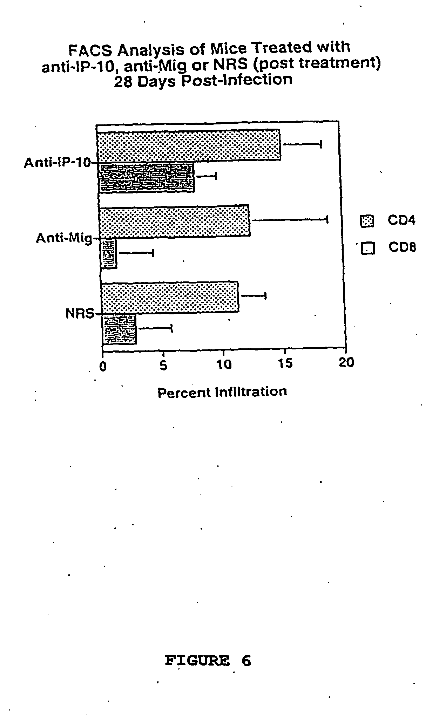

Post treatment infiltration of CD4 and CD8 T lymphocytes into the CNS of mice treated with anti-IP-10, anti-Mig or NRS was measured at 28 days post infection by FACS analysis as described above. As shown in Figure 6, anti-IP-10 treatment of MHV infected mice starting at day 12 and ending at day 19 post infection resulted in no significant differences in CD4⁺ or CD8⁺ infiltration into the CNS compared to NRS treatment (Figure 6).

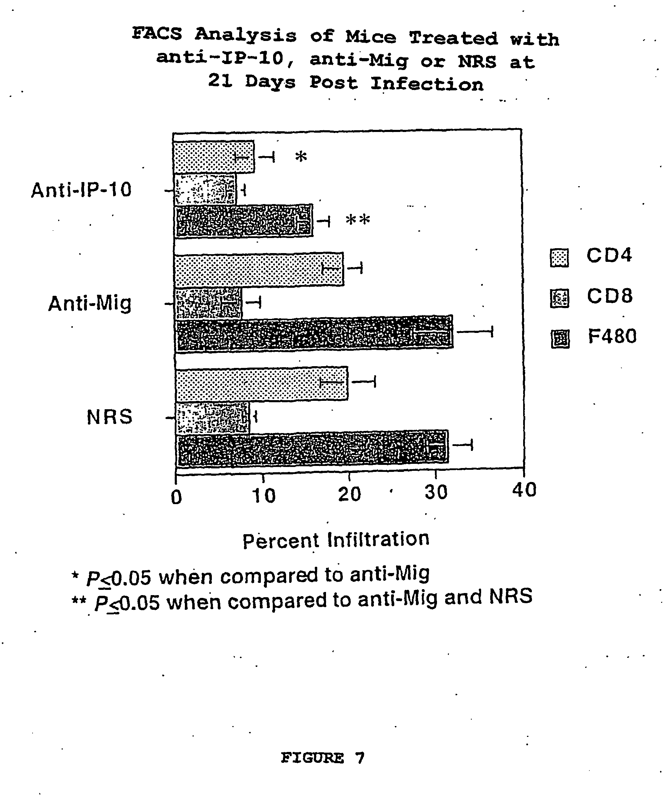

Post treatment CNS infiltration of CD4⁺ T lymphocytes, CD8⁺ T lymphocytes and macrophages was measured at day 21 post infection in mice treated with anti-IP-10, anti-Mig or NRS starting at day 12 post infection and ending at day 19 post infection via FACS analysis as described above. As shown in Figure 7, anti-IP-10 treatment of MHV infected mice starting at day 12 and ending at day 19 post infection resulted in a significant decrease in infiltration of macrophages into the CNS at 21 days post infection compared to NRS treatment. Similarly, anti-IP-10 treatment of MHV infected mice starting at day 12 and ending at day 19 post infection resulted in a significant decrease in infiltration of CD4⁺ cells into the CNS at 21 days post-infection compared to NRS treatment. Macrophages are designated F480 in Figure 7, based on the antigenic marker F4/80 used for their detection.

Demyelination of CNS nerve fibers in MHV infected mice treated with anti-IP-10, anti-Mig or NRS starting at day 12 and ending at day 19 post infection was measured at 21 days post infection. Demyelination was assessed by examination of spinal cords from animals within selected experimental groups. Tissue was prepared and the severity of demyelination was assessed as follows: Spinal cords were removed from mice at scheduled time points and fixed overnight in 10% normal buffered formalin. Tissues were then embedded in paraffin. Subsequently, sections were stained with both hematoxylin and eosin to detect inflammation and luxol fast blue to detect areas of demyelination. Following the staining, the slides were blinded and independently read by three investigators. Scoring was based on a recently described scale (Houtman and Fleming, supra, 1996). A score of 0 indicated normal tissue; 1 indicated mild inflammation with limited white matter destruction; 2 represented a moderate increase in both inflammation and demyelination; 3 represented a pronounced increase in both inflammatory lesions and demyelination; and 4 indicated numerous inflammatory/demyelinating lesions. Scores were averaged between the three investigators and presented as the mean + standard error of the mean (SEM).

As shown in Figure 8, anti-IP-10 treatment from day 12 until day 19 post infection resulted in a significant reduction in demyelination at day 21 post infection compared to NRS treatment. This reduction in demyelination corresponds to a reduction in F480 cell infiltration and CD4 cell infiltration into the CNS of MHV infected mice treated with anti-IP-10 from day 12 until day 19 post infection at 21 days post infection compared to NRS treatment.

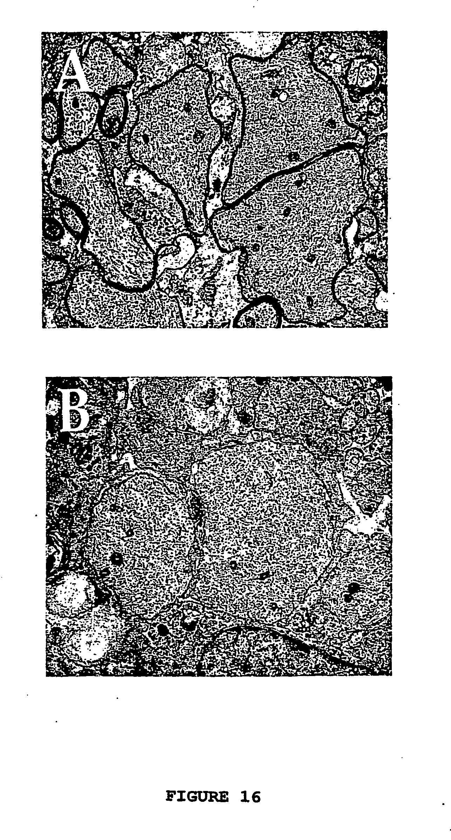

To determine whether the significant reduction in demyelination detected in mice treated with anti-IP-10 was accompanied by a gross increase in remylination light microscopic analysis of spinal cord sections of anti-IP-10, anti-Mig and NRS treated MHV infected mice was performed. Remyelination was assessed by light microscopic analysis of toluene blue stained spinal cord sections (coronal) obtained at day 21 post-infection. The significant reduction in demyelination detected in mice treated with anti-IP-10 at day 21 post-infection described above (see Figure 8) was accompanied by a gross increase in remyelination. Neither mice treated with NRS or anti-Mig experienced remyelination. The results of the light microscopic analysis indicate that the anti-IP-10 mediated neutralization of CD4 and macrophage entry into the CNS not only prevents further demyelination, but also allows repair of the damaged myelin. Figure 16 shows electron photomicrographs of axons corresponding to MHV infected mice. As shown in Figure 16A, anti-IP-10 treatment led to remyelination of viral induced demyelination as indicated by the characteristically thin dark lines surrounding each of the axons. In contrast, Figure 16B shows untreated axons with no surrounding myelin as a result of viral-induced demyelination.

### EXAMPLE II

### Selective Inhibition of Mig with Anti-Mig Antibody

This example shows selective inhibition of Mig in mice with an anti-Mig polyclonal antibody, which resulted in increased mortality during the acute phase of MHV infection, correlating with an increased viral burden on the CNS. In addition, the results described below indicate that Mig is important during the acute phase of the disease, but does not contribute to the pathology of chronic demyelination. Furthermore, this example shows that antibody mediated neutralization of Mig activity results in a shift from a protective Th1 to a Th2 response. This shift in host response is characterized by a significant reduction in CD4+ and CD8+ T cell infiltration into the CNS, decreased IFN-γ expression and increased levels of the anti-inflammatory Th2 cytokine IL-10 compared to levels found in NRS treated mice.

The mice were infected with MHV and the brains and spinal cords were isolated as described above. Rabbit polyclonal anti-Mig antibody was produced by Biosynthesis (Lewisville, TX) using a synthetic peptide CISTSRGTIHYKSLK, coupled to carrier protein KLH selected from the Mig protein sequence as described in Tannenbaum et al., *supra,* 1998. These reagents have previously been shown to be specific to Mig and not cross react with IP-10, RANTES, or other chemokines (see Tannenbaum et al., supra, 1998). MHV-infected mice were divided into two groups and treated with either NRS or anti-Mig. Mice were injected with 0.5 ml of antibody at a concentration of 0.5 mg per ml on days 0, 2, 5, 7, and 9 post infection and sacrificed at days 7 and 10 post infection.

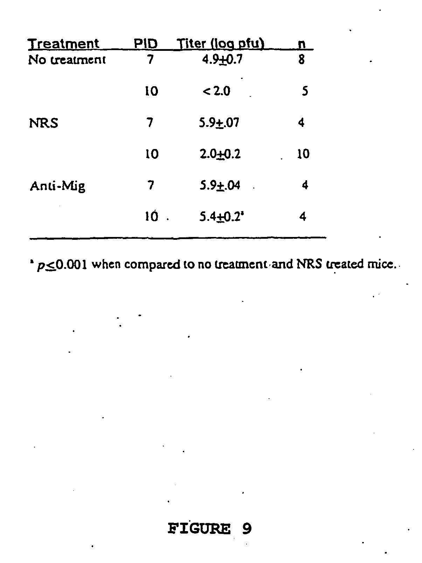

As shown in Figure 9, surviving anti-Mig treated mice displayed a 2-log increase in viral titers in the brain as compared to titers present in NRS-treated mice at day 10 post infection, which is similar to the kinetics of viral clearance shown for anti-IP-10 treatment in Figure 2 and described above. During the acute stage of the disease which lasts until day 7 post infection, virus replicates within neurons as well as glial cells (see Houtman and Fleming, supra, 1996; Dalziel, supra, 1985; and Fazackerly et al, Virology 187:178-188 (1992)). Animals that survive acute disease often develop a chronic demyelinating disease characterized by viral persistence within astrocytes and oligodendrocytes accompanied by mononuclear cell infiltration and myelin destruction (see Houtman and Fleming, supra, 1996; Buchmeier and Lane, supra, 1999).

To determine the role of Mig in the course of the MHV infection, total RNA was isolated from brains at days 7, 12 and 35 post infection and the kinetics of Mig and MHV viral gene expression were determined by ribonuclease protection assay (RPA). Total RNA was extracted from brains and spinal cords of MHV-infected animals treated with either anti-Mig or NRS as described in Lane et al., *supra*, 1998. The antisense riboprobe used to detect Mig mRNA was derived by RT-PCR amplification of cDNA generated from total RNA isolated from the brain of an MHV-infected mouse at day 7, 12 and 35 post infection. The following oligonucleotide primers were used for Mig amplification: (Forward) 5' CGT CGT CGT TCA AGG AAG and (Reverse) 5' TCG AAA GCT TGG GAGGTT. PCR amplification was performed using an automated Perkin-Elmer (Norwalk, CT) model 480 DNA thermocycler with the following profile: step 1, initial denaturation at 94°C for 30 seconds; step 2, annealing at 60°C for 30 seconds; and step 3, extension at 72°C for 45 seconds. Steps 1 through 3 were repeated 34 times for a total of 35 cycles and followed by a 7 minute incubation at 72°C. The expected PCR amplicon of 599 base pairs was cloned into the pCR Script SK+ vector (Stratagene, San Diego, CA) and sequence analysis identified over 95 percent nucleotide identity with mouse Mig (see Vanguri and Farber, J. Biol. Chem. 265(25):15049-15057(1990).

For analysis of Mig and MHV viral gene expression, the Mig riboprobe was used in combination with a riboprobe specific for the MHV spikegene previously described in Lane et al., J. Virol. 71:2202-2210 (1997). L32 was added as an internal control to verify consistency in RNA loading and assay performance as previously described in Lane et al., supra, 1998; and Lane et al., supra, 2000. For quantification of signal intensity, autoradiographs were scanned and individual chemokine bands were normalized as the ratio of band intensity to the L32 control as previously described in Lane et al., supra, 1998; and Lane et al., supra, 2000. Analysis of the values obtained from the scanned autioradiograph was performed using NIH Image 1.61 software.

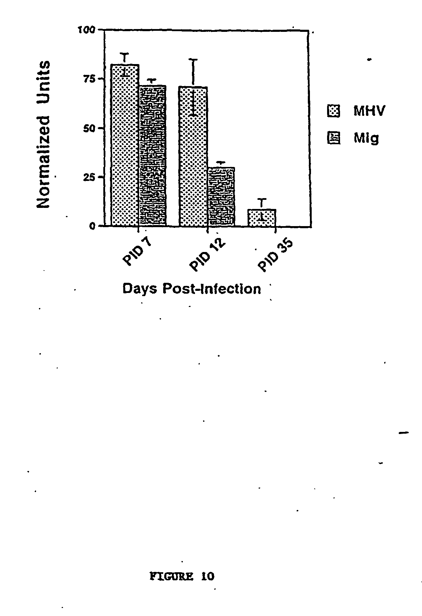

As shown in Figure 10, the Mig and MHV spike genes are expressed at days 7 and 12 post infection. However, by day 35 post infection, a time at which the animals exhibit extensive myelin loss (see Figure 8), Mig transcripts are undetectable whereas spike gene transcripts are still present. Double labeling using antisense riboprobe specific for Mig mRNA and a polyclonal antibody to glial fibrillary acidic protein (GFAP) (DAKO Corp., Carpinteria, CA) used according to manufacturer's instructions, indicate that astrocytes express Mig following MHV infection. These results indicate that Mig is important during the acute phase of the disease but does not contribute to chronic demyelination within persistently infected mice.

For studies designed to colocalize cellular antigens with in situ signal for Mig mRNA transcripts, immunohistochemical analysis preceded in situ hybridization performed as described in Lane et al, supra, 1998. The phosphate-buffered saline (PBS) used for dilution of antibodies as well as in washing steps was diethyl pyrocarbonate treated to reduce RNase contamination and loss of in situ signal. Following application of the chromagen diaminobenzidine (DAB), slides were washed twice in PBS and then prehybridized in hybridization buffer for 1 hour at 42°C. Subsequently, the linearized 35S-labeled riboprobe was added to the sections and the standard in situ hybridization procedure was performed as described in Lane et al., supra, 1998. Upon development, the slides were counterstained in hematoxylin only, then dehydrated and mounted.

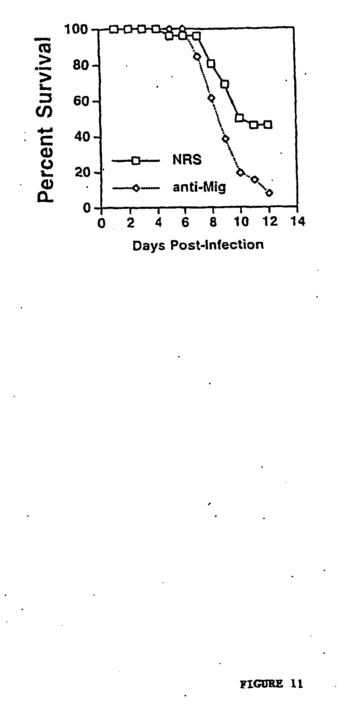

Treatment of MHV infected mice with anti-Mig antibody was performed to evaluate the functional significance of Mig expression following viral infection of the CNS. As shown in Figure 11, anti-Mig antibody treatment resulted in a marked increase in mortality as compared to control mice treated with NRS. By day 12 post infection, less than 10 percent of the anti-Mig treated mice survived the infection in contrast to 50 percent survival rate in MHV infected mice treated with NRS (Figure 11). Surviving mice treated with anti-Mig antibody displayed significantly higher (p≤ 0.001) virus titers (5.4 ± 0.3, n=4) as compared to titers present in NRS treated mice (2.0 ± 0.2, n=10) (Figure 9). These results indicate that neutralization of Mig during the acute disease results in increased mortality that correlates with an increase in viral burden within the CNS.

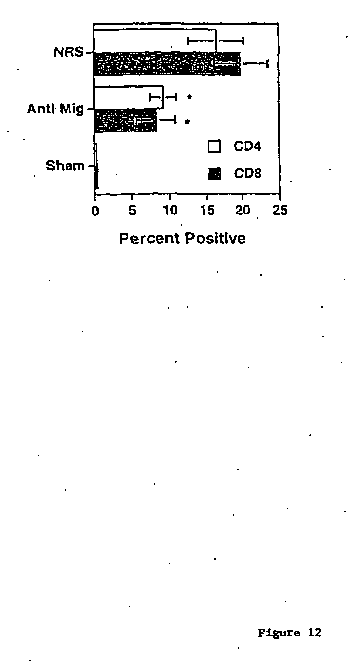

T lymphocyte isolation and flow cytometry was performed to determine the effect of anti-Mig treatment of MHV infected mice on T lymphocyte infiltration into the CNS as described above and in Lane et al., supra, 2000. Fluorescent Activated Cell Sorter (FACS) analysis at day 7 post infection indicated that MHV-infected mice treated with anti-Mig displayed significantly lower (p ≤ 0.005) levels of both CD4⁺ and CD8⁺ T lymphocyte infiltration into the CNS when compared to levels present within MHV infected mice treated with NRS (Figure 12). As shown in Figure 12, anti-Mig treatment of mice resulted in a 45 percent reduction in infiltrating CD4⁺ cells and a 60 percent reduction infiltrating CD8⁺ cells when compared to NRS treated mice (*p ≤ 0.005).

One mechanism by which T cells contribute to host defense against MHV infection of the CNS is through the release of the anti-viral cytokine IFN-γ (Pearce, supra, 1997; Lane, supra, 1997; Parra et al., J. Immunol. 162:1641-1647 (1999). To determine whether IFN-γ levels were altered in MHV infected mice treated with anti-Mig, cytokine mRNA transcript levels within the CNS of ant-Mig or NRS treated mice were evaluated by RPA at day 7 post infection.

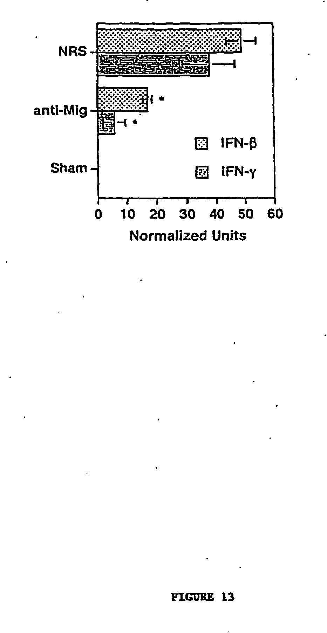

Figure 13 shows densitometric analysis of IFN-γ and IFN-β transcript levels in brains of MHV infected mice treated with either anti-Mig or NRS at day 7 post infection. The data shown in Figure 13 is presented as normalized units representing ratio of signal intensity of either IFN-γ or IFN-β to internal L32 control included in the probe set. Values were obtained from the scanned autoradiograph using NIH Image 1.61 software as described in Lane et al., supra, 1998 and Lane et al., supra, 2000. The signal intensities indicated that anti-Mig treated mice displayed an 86.7 percent reduction in IFN-γ (p ≤ 0.03) as well as a 65.1 percent reduction in IFN-β (p ≤ 0.02) transcript levels as compared to mice treated with NRS (Figure 13).

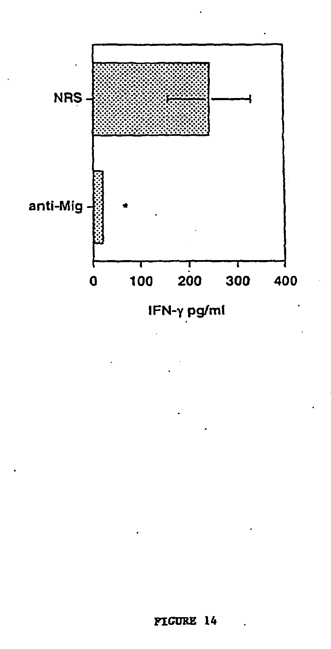

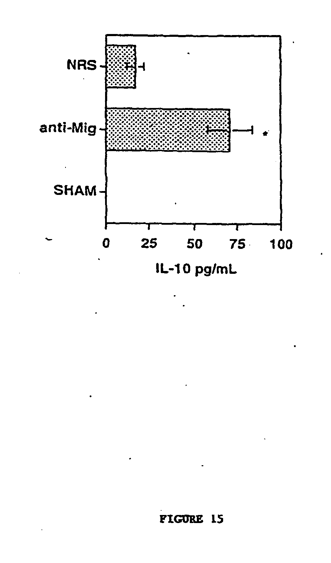

In addition, IFN-γ and IL-10 protein levels within groups were determined using the Quantikine M mouse IFN-γ or IL-10 immunoassay kit (R&D Systems, Minneapolis, MN) at day 7 post infection. Tissue samples were homogenized in 1 ml of sterile phosphate buffered saline and spun at 400 x g for 5 minutes at 4°C. Duplicate supernatant samples were used to determine respective protein levels present within the tissues according to the manufacturer's instructions. Following the enzymatic color reaction, samples were read at 450nm and respective protein levels were quantified in comparison to a standard curve supplied by the manufacturer. The results in Figures 14 and 15 are presented as picograms per milliliter. The limit of sensitivity of protein detection was approximately 8.0pg/ml. The reagents used do not cross react with other mouse cytokines.

Consistent with the RPA results, IFN-γ protein levels were reduced by 91 percent (p ≤ 0.05) within the CNS of anti-Mig treated mice as compared to NRS-treated mice as determined by ELISA (Figure 14). As shown in Figure 15, a four-fold increase in levels of the anti-inflammatory TH2 cytokine IL-10 within the CNS coincided with decreased IFN-γ levels in mice treated with anti-Mig antibody.

### EXAMPLE III

### Confirmation of IP-10 And Mig Activity by In-Vitro Assays

This example shows confirmation of IP-10 and Mig activity by *in vitro* methods as well as confirmation of neutralizing activity of a human monclonal antibody against IP-10. The assays described below are useful to identify a neutralizing agent specific for IP-10 or Mig.

Briefly, the activity of IP-10 or Mig will be neutralized with a neutralizing agent of the invention, for example, a human monoclonal antibody and the effect of this treatment on T cell migration into the central nervous system will be determined. To this end, the chemotaxis assay will utilize a human cell line that has been stably transfected with CXCR3 human receptor cDNA and characterized. Briefly, human CXCR3 receptor cDNA was cloned by RT-PCR from PHA/IL-2 activated human T cells and RNA prepared. Subcloned cDNA was subsequently sequenced to confirm PCR primer specificity. Jurkat cells were electroporated with with 20ug of the cDNA construct. Neomycin-resistant cells were cloned by limiting dilution and screened by Northern blot for expression. Subsequently, flow cytometric analysis using antihuman CXCR3 receptor antibody (Leukocyte, Inc., Cambridge, MA) confirmed receptor expression.

Chemotaxis will be assessed using a well-established protocol described in Luster et al., Proc. Assoc. Am Physicians 110(3):183-196 (1998). Briefly, chemotaxis will utilize 48-well chemotaxis chambers (Neuroprobe, Cabin John, MD) using polyvinylpyrrolidone-free polycarbonate membranes with 5 micron pores. The membranes will be submerged in a 2ug/ml solution of human collagen type IV (Collaborative Biomedical Products, Beford, MA) and incubated at 37°C for 1 hr prior to use. Human recombinant proinflammatory molecule IP-10 (PeproTech, Rocky Hill, NJ) or Mig will be incubated either with or without the compounding human mAb and receptor-expressing T cells at a concentration of 5x10⁴/well and obtained from peripheral bleed followed by Ficoll-Hypaque gradient spin. Activated cells will migrate in response to active proinflammatory molecule and cells will be stained with Diff-Quick (Baster, Valencia, CA) and counted in 4 randomly high-power fields. Non-transfected cells will be used as negative controls.

A second assay is based on detection of intracellular calcium, changes in which are a hallmark of chemokine binding to a specific chemokine receptor. Intracellular calcium ([Ca²⁺]i) in single, receptor-expressing T cells as described above will be determined using a previously described protocol described in Luster et al., Proc. Assoc. Am Physicians 110(3):183-196 (1998). Cells (1x10⁵/ml) will be loaded with fura-2/AM at 2mM at 37°C for 30 min in PIPES buffer. One ml of cells will be in a special chamber (Nunc, Roskilde, Denmark) without any coating of chamber to eliminate background signaling. Changes in [Ca²⁺]i will be determined using a digital imaging system with a light-sensitive camera. Recombinant human proinflammatory molecule will be incubated with or without mAb at different concentration and added to separate chambers of cells. The [Ca²⁺]i will be determined before and after stimulation.

## Claims

1. Use of a neutralizing agent specific for interferon inducible protein of 10 kDa (IP-10) for the preparation of a medicament for promoting remyelination in a subject affected with a demyelinating disease at a chronic stage, wherein the IP-10 neutralizing agent is selected from the group consisting of an antibody, antibody fragment or immunoadhesion against IP-10, an antisense nucleic acid to IP-10, a ribozyme specific to IP-10, and a fragment or peptidomimetic of the CXCR3 receptor that binds IP-10.

2. Use of a neutralizing agent specific for interferon inducible protein of 10 kDa (IP-10) for the preparation of a medicament for reducing the severity of a demyelinating disease in a subject having a demyelinating disease at a chronic stage, comprising remyelinating demyelinated nerve fibers and impeding demyelination of said nerve fibers, wherein the IP-10 neutralizing agent is selected from the group consisting of an antibody, antibody fragment or immunoadhesion against IP-10, an antisense nucleic acid to IP-10, a ribozyme specific to IP-10, and a fragment or peptidomimetic of the CXCR3 receptor that binds IP-10.

3. The use of claim 1 or 2, wherein said subject is a mammal.

4. The use of claim 3, wherein said subject is a human.

5. The use of claims 1 to 4, wherein said demyelinating disease is multiple sclerosis (MS).

6. The use of claims 1 to 5, wherein said neutralizing agent specific for interferon inducible protein of 10 kDa (IP-10) is administered by intravenous administration.

## Patentansprüche

1. Verwendung eines neutralisierenden Agens, welches spezifisch für Interferon-induzierbares Protein mit 10 kDa ist (IP-10) zur Herstellung eines Medikaments zur Förderung der Remyelinierung bei einem Subjekt, welches an einer demyelinierenden Erkrankung in einem chronischen Stadium leidet, wobei das IP-10 neutralisierende Agens ausgewählt ist aus der Gruppe bestehend aus einem Antikörper, Antikörperfragment oder Immunadhäsion gegen IP-10, einer Antisense Nukleinsäure gegen IP-10, einem für IP-10 spezifischen Ribozym und einem Fragment oder Peptidomimetikum des CXCR3 Rezeptors, das an IP-10 bindet.

2. Verwendung eines neutralisierenden Agens, welches spezifisch für Interferon-induzierbares Protein mit 10 kDa (IP-10) ist, zur Herstellung eines Medikaments zur Reduktion der Ernsthaftigkeit einer demyelinierenden Erkrankung bei einem Subjekt mit einer demyelinierenden Erkrankung in einem chronischen Stadium, umfassend die Remyelinierung von demyelinierten Nervenfasern und die Hemmung der Demyelinierung der Nervenfasern, wobei das IP-10 neutralisierende Agens ausgewählt ist aus der Gruppe bestehend aus einem Antikörper, Antikörperfragment oder Immunadhäsion gegen IP-10, einer Antisense Nukleinsäure gegen IP-10, einem für IP-10 spezifischen Ribozym und einem Fragment oder Peptidomimetikum des CXCR3 Rezeptors, das an IP-10 bindet.

3. Die Verwendung nach Anspruch 1 oder 2, **dadurch gekennzeichnet, dass** das Subjekt ein Säuger ist.

4. Die Verwendung nach Anspruch 3, **dadurch gekennzeichnet, dass** das Subjekt ein Mensch ist.

5. Die Verwendung nach Ansprüchen 1 bis 4, **dadurch gekennzeichnet, dass** die demyelinierende Krankheit Multiple Sklerose (MS) ist.

6. Die Verwendung nach Ansprüchen 1 bis 5, **dadurch gekennzeichnet, dass** das neutralisierende Agens, welches spezifisch für Interferon-induzierbares Protein mit 10 kDa (IP-10) ist, durch intravenöse Verabreichung verabreicht wird.

## Revendications

1. Utilisation d'un agent neutralisant spécifique d'une protéine de 10 kDa (IP-10) inductible par interféron pour la préparation d'un médicament pour l'amélioration de la remyélinisation chez un sujet affecté d'une maladie démyélinisante à un stade chronique, dans laquelle l'agent neutralisant IP-10 est choisi dans le groupe constitué d'un anticorps, d'un fragment d'anticorps ou d'une immunoadhésion contre IP-10, un acide nucléique antisens de IP-10, un ribozyme spécifique de IP-10, et un fragment ou un peptidomimétique du récepteur CXCR3 qui fixe IP-10.

2. Utilisation d'un agent neutralisant spécifique d'une protéine inductible par interféron de 10 kDa (IP-10) pour la préparation d'un médicament afin de réduire la gravité d'une maladie démyélinisante chez un sujet ayant une maladie démyélinisante à un stade chronique, comprenant la remyélinisation des fibres nerveuses démyélinisées et pour empêcher la démyélinisation desdites fibres nerveuses, dans laquelle l'agent neutralisant IP-10 est choisi dans le groupe constitué d'un anticorps, d'un fragment d'anticorps ou d'une immunoadhésion contre IP-10, un acide nucléique antisens de IP-10, un ribozyme spécifique de IP-10, et un fragment ou un peptidomimétique du récepteur CXCR3 qui fixe IP-10.

3. Utilisation selon la revendication 1 ou 2, dans laquelle ledit sujet est un mammifère.

4. Utilisation selon la revendication 3, dans laquelle ledit sujet est un humain.

5. Utilisation selon les revendications 1 à 4, dans laquelle ladite maladie démyélinisante est la sclérose multiple (MS).

6. Utilisation selon les revendications 1 à 5, dans laquelle ledit agent neutralisant spécifique d'une protéine de 10 kDa (IP-10) induite par interféron est administré par voie intraveineuse.
